(19) Europäisches Patentamt
European Patent Office
Office européen des brevets

(11) EP 0 845 273 B1

(12) EUROPÄISCHE PATENTSCHRIFT

(45) Veröffentlichungstag und Bekanntmachung des Hinweises auf die Patenterteilung:
25.05.2005 Patentblatt 2005/21

(51) Int Cl.⁷: A61M 1/16

(21) Anmeldenummer: 97120890.5

(22) Anmeldetag: 28.11.1997

(54) **Vorrichtung zur in-vivo-Bestimmung von Parametern der Hämodialyse**

Means for in vivo determining haemodialysis parameters

Dispositif de détermination in vivo de paramètres d'hémodialyse

(84) Benannte Vertragsstaaten:
DE ES FR GB IT

(30) Priorität: 30.11.1996 DE 19649776

(43) Veröffentlichungstag der Anmeldung:
03.06.1998 Patentblatt 1998/23

(73) Patentinhaber: Fresenius Medical Care
Deutschland GmbH
61352 Bad Homburg v.d.H. (DE)

(72) Erfinder: Goldau, Rainer
97440 Schraudenbach (DE)

(74) Vertreter: Luderschmidt, Schüler & Partner GbR
Patentanwälte,
John-F.-Kennedy-Strasse 4
65189 Wiesbaden (DE)

(56) Entgegenhaltungen:
EP-A- 0 428 927          WO-A-95/32010

**Beschreibung**

Die Erfindung bezieht sich auf eine Vorrichtung zur in-vivo-Bestimmung von Parametern der Hämodialyse entsprechend dem Oberbegriff des Anspruches 1.

Die Hämodialyse wird seit einer Vielzahl von Jahren erfolgreich zur Behandlung von nierenkranken Patienten verwendet und hat sich weltweit bewährt.

Die Nieren eines Menschen haben mehrere Funktionen, zum Beispiel die Ausscheidung von Wasser, die Entfernung von Stoffwechselabfallprodukten (Harnstoff, Kreatinin) und die Beteiligung an der Einstellung der Konzentration verschiedener Stoffe wie der Elektrolyte des Blutes (Natrium, Bicarbonat, usw.) auf bestimmte Werte.

Die Hämodialyse ist ein Behandlungsverfahren zur Kompensation von Fehlfunktionen der Nieren bezüglich der Entfernung von Stoffwechselabfallprodukten und der Beteiligung an der Einstellung von Elektrolytkonzentrationen im Blut.

Dieses Behandlungsverfahren wird mit einem Dialysator durchgeführt, welcher praktisch ein Austauscher mit zwei über eine semipermeable Membran voneinander getrennten Kammern ist, einer Blutkammer zum Anschluß an einen extrakorporalen Blutkreislauf und einer Kammer für die Dialysierflüssigkeit, welche mit einem Behälter für Dialysierflüssigkeit in einem Dialysatkreis verbunden ist. Eine klassische Dialysierflüssigkeit enthält dabei die hauptsächlichen Blutelektrolyte in der Konzentration nahe den Konzentrationen des Blutes eines Gesunden.

Während einer Behandlung wird das Blut des Patienten und die Dialysierflüssigkeit an beiden Seiten der Membran i.a. im Gegenstrom mit einer vorgegebenen Flußrate vorbeigeführt. Die Ausscheidungsprodukte des Stoffwechsels diffundieren durch die Membran von der Blutkammer zur Kammer für Dialysierflüssigkeit, während die gleichzeitig im Blut und in der Dialysierflüssigkeit vorhandenen Elektrolyte von der Kammer höherer Konzentration zur Kammer niedriger Konzentration diffundieren. Durch Anlegen eines Transmembrandruckes kann der Stoffwechsel zusätzlich beeinflußt werden (Ultrafiltration).

Um das Behandlungsverfahren optimieren zu können, ist die in-vivo-Bestimmung von Parametern der Hämodialyse, also während der Durchführung der Behandlung, notwendig. Ein solcher Parameter ist insbesondere der Wert für die Austauschleistung des Dialysators, dargestellt durch die sogenannte "Clearance" bzw. "Dialysance D". Es sind dabei folgende Definitionen üblich:

Die Clearance für einen bestimmten Stoff K bezeichnet nach DIN 58 352, Teil 1, dasjenige virtuelle (errechnete) Blutvolumen, das pro Minute durch den Dialysator vollkommen von diesem Stoff befreit wird.

Die Dialysance ist ein weiterer Begriff zur Bestimmung der Leistungsfähigkeit eines Dialysators, bei dem auch die Konzentration des am Stoffaustausch im Dialysator beteiligten Stoffes in der Dialysierflüssigkeit berücksichtigt wird.

Neben diesen Leistungsdaten des Dialysators sind auch andere Parameter von Bedeutung, insbesondere die Werte des wässrigen Anteils des Blutes (des effektiven Blutflusses), des Hämatokrits und der Bluteingangskonzentration.

Die meßtechnisch-mathematische Quantifizierung der Blutreinigungsverfahren und in Verbindung damit die Bestimmung der vorgenannten Parameter der Dialyse ist relativ komplex. Es wird insoweit beispielhaft auf das Werk von H.E. Franz "Blutreinigungsverfahren", erschienen im Georg Thieme Verlag Stuttgart, New York 1990, insbesondere Seite 479-492, Bezug genommen.

Danach ergibt sich insbesondere für die Bestimmung der Dialysance bzw. der Clearance für einen gegebenen Elektrolyten, beispielsweise Natrium als Stoff, bei der Ultrafiltration = O, folgendes. Die Dialysance D ist gleich dem Verhältnis zwischen dem blutseitigen Massentransport für diesen Elektrolyten $Qb \times (cbi-cbo)$ und der Konzentrationsdifferenz dieses Elektrolyten zwischen dem Blut und der Dialysierflüssigkeit am jeweiligen Eingang des Dialysators $(cbi-cdi)$.

$$D = Qb\frac{(cbi - cbo)}{cbi - cdi} \tag{1}$$

Aus Gründen der Massenbilanz (die aus dem Blut entfernte Stoffmenge ist gleich der in derselben Zeit im Dialysat abtransportierten Stoffmenge) gilt

$$Qb \cdot (cbi-cbo) = -Qd \cdot (cdi-cdo) \tag{2}$$

Daraus folgt aus (1) und (2) für die Dialysance dialysatseitig:

$$D = -Qd\frac{(cdi - cdo)}{cbi - cdi} \tag{3}$$

Dabei bedeuten in (1) bis (3):

Qb = effektiver Blutfluß
Qd = Dialysierflüssigkeitsfluß
cb = Konzentration des Stoffes im Lösungsvolumen des Blutes
cd = Konzentration des Stoffes in der Dialysierflüssigkeit
i = Eingang des Dialysators
o = Ausgang des Dialysators

Der effektive Blutfluß ist der Fluß des Blutanteils im Vollblutfluß, in dem die zu entfernenden Stoffe gelöst sind, d.h. er bezieht sich auf das komplette (wäßrige) Lösungsvolumen für diesen Stoff. Je nach Stoff kann das der Plasmawasserfluß oder der Blutwasserfluß, d.h. der gesamte Wasseranteil am Vollblut sein.

Für den Fall eines besonderen Stoffwechselausscheidungsprodukts (zum Beispiel Harnstoff) ist cdi = O, und man spricht dann nicht mehr von der Dialysance, sondern von der Clearance K für dieses Stoffwechselprodukt.

$$K = Qb\frac{(cbi - cbo)}{cbi} = Qd\frac{cdo}{cbi}$$

Alle bekannten Verfahren zur in-vivo-Bestimmung von Parametern der Hämodialyse setzen bei diesen Überlegungen ein. Den meisten von ihnen ist das Bestreben gemeinsam, ohne einen direkten Meßeingriff auf der Blutseite auszukommen, weil dies eine nicht unerhebliche Gefahrenquelle darstellen könnte. Es besteht daher das Bestreben, die zu bestimmenden Größen von Meßwerten allein aus dialysatseitigen Messungen abzuleiten, auch was die blutseitigen Größen anbelangt. Eine gängige Basismethode ist dabei, daß man stromauf und stromab des Dialysators die Stoffkonzentration in der Dialysierflüssigkeit mißt und daraus den dialysatseitigen Massentransport Qd x (cdi - cdo) bestimmt, und aus diesen Werten über o. a. Gleichungen andere Größen ableitet, insbesondere den Wert für die Bluteingangskonzentration cbi, der mit in die Gleichungen als mathematische Unbekannte eingeht. Es müssen dabei nicht zwingend beide Werte cdi und cdo gemessen werden. Der Eingangswert cdi kann auch definiert in der frischen Dialysierflüssigkeit eingestellt werden.

Wenn insbesondere der Wert cbi eines Elektrolyten bestimmt werden soll, können cdi und cdo durch Leitfähigkeitsmessungen ermittelt werden. Im Fall von NaCl ist sogar eine unspezifische Messung ausreichend, da NaCl den dominierenden Anteil an der Leitfähigkeit der beteiligten Flüssigkeiten hervorruft. Diese Basismethode ist durch die EP 0 097 366 bekannt geworden.

Die einzelnen bekannten Verfahren unterscheiden sich ausgehend von der durch die vorstehende Vorrichtung durchgeführten Basismethode in der Meß- und Auswertemethodik. Sie sollen im folgenden näher erläutert werden.

Durch die EP 0 291 421 B1 ist ein Verfahren zur Bestimmung der Bluteingangskonzentration bekannt geworden, bei dem rampenförmig die Dialysateingangskonzentration verändert wird, um den Punkt zu bestimmen, wo kein Elektrolyttransfer über die Membran mehr stattfindet. Das bekannte Verfahren arbeitet daher nach dem Prinzip, die Eingangsleitfähigkeit der Dialysierflüssigkeit soweit zu verändern, daß sie sich nicht mehr von der Ausgangsleitfähigkeit unterscheidet. Dann muß sie die Bluteingangsleitfähigkeit angenommen haben (cbi = cdi). Auf der Basis der Gleichungen (1) bis (3) lassen sich dann andere Prameter der Hämodialyse ableiten. Nachteilig bei diesem Verfahren ist die verhältnismäßig lange Meßzeit, bedingt durch die Zeitspanne bis zum Erreichen des stabilen Gleichgewichtszustandes beim Einstellen der Dialysierflüssigkeit auf den neuen Eingangskonzentrationswert, der sich zudem nicht sofort an jedem Punkt des Dialysators auswirkt. Es dauert systembedingt eine gewisse Zeit, bis ein Leitfähigkeitssprung am Dialysateingang zu stabilen Verhältnissen am Dialysatausgang führt. Die zum Erreichen des stabilen Gleichgewichtszustandes erforderliche Zeitspanne ist dabei im wesentlichen bestimmt von der Größe der Leitfähigkeitsveränderung pro Zeit. Innerhalb dieser langen Zeitspanne können sich jedoch Parameter der Dialyse ändern und somit den zu bestimmenden Wert verfälschen. Insbesondere ist zu beachten, daß das vorgenannte bekannte Verfahren (wie alle anderen auch) direkt die Bluteingangskonzentration cbi durch den herbeigeführten Elektrolyttransfer verändern kann. Im bekannten Fall ist dieser systematische Fehler durch die Art der Veränderung der Konzentration auf der Dialysatseite besonders groß. Das bekannte Verfahren führt damit nicht zu genauen Meßwerten für die in-vivo zu bestimmenden Parameter der Hämodialyse. Hinzu kommt, daß eine relativ aufwendige zusätzliche Vorrichtung zum Variieren der Dialysateingangskonzentration benötigt wird.

Ein weiteres Verfahren zur in-vivo-Bestimmung von Parametern der Hämodialyse ist durch die DE 39 38 662 C2 (= EP 0 428 927 A1) bekannt geworden. Bei diesem Verfahren wird der Dialysat-Elektrolyttransfer jeweils bei zwei unterschiedlichen Dialysat-Eingangskonzentrationen gemessen. Auf der Basis der Gleichung (3) für die beiden Fälle und der Annahme der Konstanz von cbi kann dann die Dialysance bestimmt werden, indem die Differenz zwischen den Differenzen der Dialysierflüssigkeits-Ionenkonzentration an der Eingangsseite und der Ausgangsseite des Dialysators zum Zeitpunkt der ersten und der zweiten Messung bestimmt wird, diese durch die Differenz der Dialysierflüssigkeits-Ionenkonzentration an der Eingangsseite zum Zeitpunkt der ersten Messung und der zweiten Messung geteilt wird und der Quotient hieraus mit dem Dialysierflüssigkeitsfluß multipliziert wird.

Bei diesem Verfahren muß ebenfalls angenommen werden, daß die Bluteingangskonzentration cbi bei beiden Messungen unverändert bleibt, da sonst das entsprechende Gleichungssystem nicht nach der Bluteingangskonzentration cbi unter Bestimmung der Dialysance aufgelöst werden könnte.

Eine Erhöhung der Dialysat-Eingangskonzentration cdi erhöht jedoch auch die Bluteingangskonzentration. Weiterhin wird bei dem bekannten Verfahren die Annahme konstanter Dialysance bei veränderten Eingangs- und Ausgangsleitfähigkeiten gemacht. Über die Grenzen der Gültigkeit dieser Annahme sind jedoch keine verifizierbaren Angaben bekannt. Wie alle Verfahren mit Änderung der Dialysat-Eingangskonzentration benötigt auch dieses Verfahren eine zusätzliche Vorrichtung zum Variieren der Dialysat-Eingangskonzentration.

In einer Weiterbildung des bekannten Verfahrens ist auch vorgesehen, zusätzlich die Abhängigkeit der ermittelten Parameter, z.B. der Dialysance, vom Dialysierflüssigkeitsfluß zu bestimmen. Dazu wird der Dialysierflüssigkeitsfluß auf unterschiedliche Werte eingestellt und jeweils die Dialysance für jeden der Flußwerte auf der Basis der Messung des Dialysat-Elektrolyttransfers bei zwei unterschiedlichen Dialysat-Konzentrationen gemessen.

Durch die EP 0 330 892 A 1 sowie durch die daraus ausgeschiedene EP 0 547 025 ist ein weiteres einschlägiges Verfahren bekannt geworden, das hauptsächlich die Bestimmung der Bluteingangskonzentration cbi zum Ziel hat.

In diesem Zusammenhang ist auch eine Methode zur Ermittlung der relativen Dialysance D/Qd angegeben, bei der die Differenz der Dialysierflüssigkeits-Ionenkonzentration an der Ein- und Ausgangsseite des Dialysators, d.h. die Elektrolyttransferrate, bestimmt wird. Um D/Qd fortlaufend zu bestimmen, wird die Leitfähigkeit, d.h. die Ionenkonzentration, in der Dialysierflüssigkeit stufenweise geändert, wobei für jeden Leitfähigkeitswert jeweils die relative Dialysance durch die zugehörige Messung des Elektrolyttransfers bestimmt wird.

Dieses bekannte Verfahren arbeitet daher ebenso mit unterschiedlichen Konzentrationseinstellungen im Dialysatkreis mit den vorstehend erläuterten Nachteilen (lange Meßzeiten, aufwendige Vorrichtung usw.), zumal im bekannten Fall "die Gleichung nur aufgeht", wenn bei einer Konzentrationsänderung neben dem Abwarten hinsichtlich der Einstellung des stabilen Zustandes auf der Dialysatseite auch die Gleichgewichtssituation cbi = cdi zur vorherigen Bestimmung von cbi abgewartet wird.

In der US-A- 5,567,320 wird ein Verfahren zur Bestimmung von am Stoffaustausch beteiligten Parametern der Hämodialyse, wie Bluteingangskonzentration oder Dialysance, beschrieben, bei der die Dialysierflüssigkeit im Meßintervall auf drei unterschiedliche Konzentrationen des zu betrachtenden Stoffes bei konstantem Blut- bzw. Dialysierflüssigkeitsfluß eingestellt wird.

Auch dieses bekannte Verfahren arbeitet daher mit aufeinanderfolgenden unterschiedlichen Konzentrationseinstellungen im Dialysatkreis, mit der bereits erwähnten Folge der aufwendigen Vorrichtung, der relativ langen Meßzeit und der Rückwirkung auf die Bluteingangskonzentration.

Durch die US-A-5,110,477 ist ein Verfahren zur Bestimmung der Clearance eines Dialysators bekannt geworden, bei dem sowohl auf der Dialysat- als auch auf der Blutseite Kalibrierlösungen durch den Dialysator geleitet werden. Aufgrund von Vergleichen mit Sollwerten kann dann auf die Dialysance bzw. die Clearance geschlossen werden. Dieses Verfahren hat den Nachteil, daß es aufwendig ist und nicht "in-vivo", sondern nur "in vitro", d.h. außerhalb der laufenden Dialysebehandlung, durchgeführt werden kann.

Durch die WO 95/32010 ist ein Verfahren und eine Vorrichtung zum optimierenden Einstellen der Dialysebehandlung bekannt geworden, bei dem ein Parameter, insbesondere der Blutfluß, derart variiert wird, daß eine optimale Entfernung von Schadstoffen (= maximale Dialysance) erreicht wird. Am Dialysatausgang wird dabei die Konzentration eines Metaboliten (Harnstoff) direkt gemessen und der Blutfluß solange geändert, bis die Konzentration ein Maximum zeigt. Dieses Verfahren ist jedoch sehr zeitaufwendig und ist auf die Optimierung der Ausscheidung beschränkt.

Der Erfindung liegt die Aufgabe zugrunde, eine Vorrichtung zu schaffen, die in-vivo-Bestimmung von Parametern der Hämodialyse schneller und genauer als beim Stand der Technik durchführt.

Die Lösung dieser Aufgabe gelingt ausgehend von der eingangs bezeichneten Vorrichtung durch die kennzeichnenden Merkmale des Anspruches 1.

Gemäß der erfindungsgemäßen Vorrichtung wird nicht die Dialysateingangskonzentration, sondern der Blutfluß bzw. der Dialysatfluß variiert. Auf den ersten Blick erweckt dies zwar den Eindruck, daß es letztendlich gleich sei, welche Parameter variiert werden, damit mit Hilfe zweier Gleichungen zwei unbekannte Meßgrößen bestimmt werden können. Dies ist hier jedoch nicht der Fall: Durch die Veränderung des Blut- oder des Dialysatflusses wird auch die Dialysance verändert, d.h. das Gleichungssystem (1) bzw. (3) kann nicht mehr so einfach wie im Fall der DE 39 38 662 aufgelöst werden.

Die Erfindung bietet gegenüber den bekannten Vorrichtungen die folgenden Vorteile:

Die erfindungsgemäße Vorrichtung bedient sich zwar ebenfalls der Meßwerte für die Eingangs- und Ausgangskonzentration im Dialysat, wobei nicht beide Werte zwingend gemessen werden müssen; der Eingangswert kann auch definiert eingestellt werden. Es kann jedoch schneller realisiert werden. Die Eingangsleitfähigkeit der Dialysierflüssigkeit braucht funktionell nicht verändert zu werden. Stattdessen wird die Flußrate, also vorrichtungsmäßig die Pumpengeschwindigkeit, geändert. Dies wirkt sich im Gegensatz zur Leitfähigkeitsänderung sofort an jedem Punkt des Dialysators aus und führt zu einer beachtlichen Verkürzung der Meßzeit.

Da die Gleichgewichtsveränderungen im Dialysator nicht so gravierend sind, d.h. weil die Leitfähigkeitssprünge des Ausgangsdialysates kleiner sind, führt auch dieses Moment zu einer Verkürzung der Meßzeit.

Wegen der Verkürzung der Meßzeit fallen auch Rückkopplungen auf die Bluteingangskonzentration weniger ins Gewicht. Die Annahme einer konstanten Dialysance während der Meßzeit entfällt. Da die Dialysance flußabhängig ist, ändert sie sich und geht unmittelbar als zu betrachtende Größe in die Bestimmung des Parameters mit ein. Schließlich benötigt die Erfindung keine zusätzliche Vorrichtung zum Verändern der Ionenkonzentration am Dialysateingang des Dialysators. Es bedarf vorrichtungsmäßig lediglich einer relativ einfachen Einrichtung zum Verstellen der Drehzahl der Blut- bzw. Dialysatpumpe.

In der zum Stand der Technik gehörenden, nicht vorveröffentlichten DE 195 41 783 C1 wird ein Verfahren zur Ermittlung hämodynamischer Parameter beschrieben, die im Sachzusammenhang mit dem Fistelfluß während dieser extrakorporalen Blutbehandlung mit einem Dialysator stehen, wie der Fistelfluß selbst, die Körpertemperatur und das Herzminutenvolumen. Dazu wird eine bestimmte Kenngröße des Blutes fortlaufend gemessen, die zum Beispiel die Konzentration eines Blutbestandteils oder der Hämatokrit sein kann. Gleichzeitig wird der Blutfluß kontinuierlich zwischen zwei Grenzwerten variiert und sowohl die Flußwerte als auch die jeweils zugehörigen Meßwerte der Kenngröße als Wertepaar gespeichert. Aus der abgespeicherten Folge der Wertepaare läßt sich insbesondere eine Aussage über den Fistelfluß in bezug auf den Blutfluß und damit über die Fistelrezirkulation gewinnen.

Bei diesem Verfahren geht es jedoch nicht um die Ermittlung von am Stoffaustausch des Dialysators beteiligten Parametern auf der Basis von dialysatseitigen Messungen der Stoffkonzentration in der Dialysierflüssigkeit und zwei diskreter von zwei Blutfluß- und/oder Dialysatflußeinstellungen abgeleiteter Größen in Verbindung mit Werten, abgeleitet aus Beziehungen zwischen den Stoffaustausch im Dialysator beschreibenden Kenngrößen des Dialysators.

Gemäß einer Weiterbildung der Erfindung ist es zweckmäßig, wenn die Auswertschaltung der art ausgebildet ist, daß der Blutfluß $(Q_{vb})$ auf mindestens zwei unterschiedliche Werte eingestellt wird, von dem jeweiligen Blutfluß ein Signal für den effektiven Blutfluß $(Q_b)$ oder für eine mit $Q_b$ zumindest im Meßintervall über eine bekannte Beziehung verknüpfte Meßgröße abgeleitet wird.

Im Fall der eingangs gewürdigten WO 95/32010 ist zwar auch eine Verstellung des Blutflusses vorgesehen, jedoch dient sie dort zum iterativen Einstellen eines Meßwertes am Dialysatausgang des Dialysators, wogegen im Fall der Erfindung die unterschiedliche Einstellung der Flußraten zum Ableiten von Meßgrößen dient, die direkt in die wertmäßige Bestimmung eines Parameters der Hämodialyse eingehen.

Gemäß einer Ausgestaltung der Erfindung ist vorgesehen, daß die Auswertschaltung derart ausgebildet ist, daß zusätzlich oder alternativ der Dialysierflüssigkeitsfluß $(Q_d)$ auf mindestens zwei unterschiedliche Werte eingestellt wird und jeweils eine entsprechende Meßgröße für den Dialysierflüssigkeitsfluß abgeleitet wird. Diese Möglichkeit gibt einen weiteren Freiheitsgrad bei der Bestimmung des gewünschten Parameters der Hämodialyse. Im Fall der eingangs gewürdigten DE 39 38 662 ist zwar auch eine Verstellung des Dialysatflusses vorgesehen, jedoch wird dort der zu ermittelnde Parameter aus anderen Meßgrößen für jede Flußeinstellung erneut bestimmt, d.h., es wird aus einer Folge der Dialysatflüsse eine Folge von Werten des zu ermittelnden Parameters bestimmt, wogegen im Fall der Erfindung aus den unterschiedlichen Werten der Dialysatflüsse ein einziger Wert des zu ermittelnden Parameters bestimmt wird.

Für die Ableitung der Meßgrößen für Blut- sowie Dialysatflüsse stehen dem Fachmann eine Reihe von Möglichkeiten zur Verfügung, zum Beispiel direkt das Ausgangssignal eines Durchflußmeßgerätes oder indirekt die Drehzahl-Einstellsignale für die zugehörigen Pumpen.

Vorzugsweise wird die Einrichtung zum Bereitstellen von Dialysier flüssigkeit derart ausgebildet daß die Eingangs-Konzentration $(c_{di})$ der Dialysierflüssigkeit konstant ist. Dadurch entfallen die eingangs erläuterten, durch die Veränderungen in der Eingangskonzentration der Dialysierflüssigkeit bedingten Nachteile. Die erfindungsgemäße Vorrichtung gibt jedoch die Möglichkeit, wenn es die Bestimmung des Parameters der Hämodialyse im Einzelfall erfordert, daß im Meßintervall die Eingangs-Konzentration $(c_{di})$ der Dialysierflüssigkeit innerhalb gewisser Grenzen geändert wird.

Weitere ausgestaltende Merkmale sowie Weiterbildungen der Erfindung sind in entsprechenden Unteransprüchen enthalten.

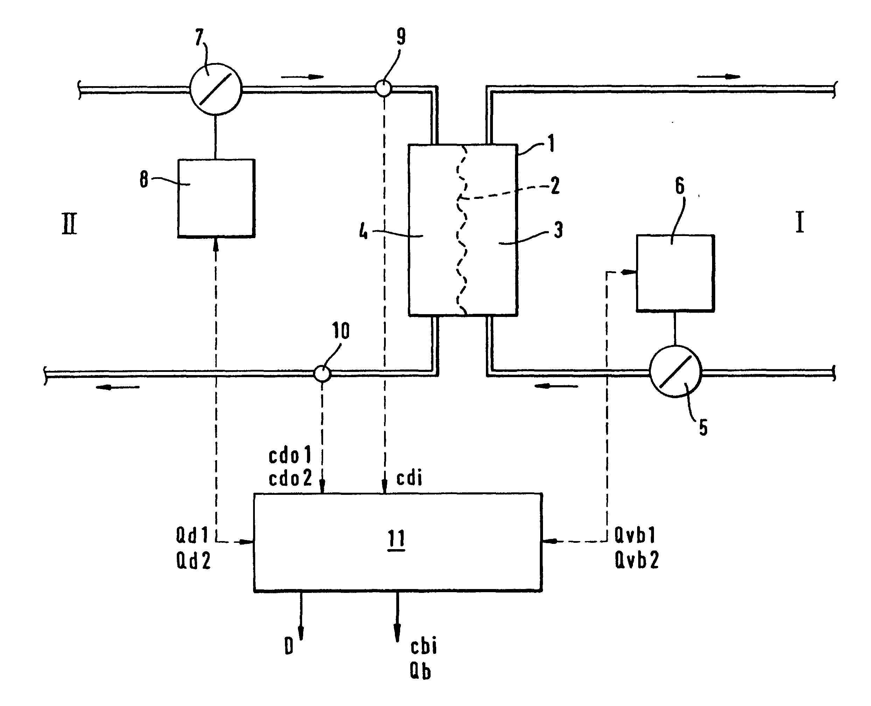

Anhand eines in der Zeichnung dargestellten Prinzipschaltbildes der erfindungsgemäßen Vorrichtung soll die Erfindung näher erläutert werden.

In der Zeichnung ist ein Prinzipschaltbild der erfindungsgemäßen Vorrichtung dargestellt.

Die Vorrichtung weist einen Dialysator 1 mit einer semipermeablen Membran 2 auf, die eine Blutkammer 3 von einer Dialysatkammer 4 trennt. Die Blutkammer 3 ist an einen extrakorporalen Kreislauf I angeschlossen, in dem das zu reinigende Blut mit einer durch eine Blutpumpe 5 vorgegebenen Flußrate fließt. Mit 6 ist eine Einrichtung bezeichnet, mittels der die Drehzahl der Blutpumpe 5 und damit der Vollblutfluß $Q_{vb}$ geändert werden kann. Solche Einrichtungen sind Stand der Technik, wie der übrige Aufbau des extrakorporalen Kreislaufes I ebenfalls Stand der Technik ist und daher in dem Prinzipschaltbild nach der Zeichnung nicht dargestellt ist. Die Dialysatkammer 4 ist an einen Dialysatkreislauf II mit konventionellem Aufbau angeschlossen, von dem der Übersicht halber nur eine Dialysatpumpe 7 mit einer zugehörigen Einrichtung 8 zum Ändern der Drehzahl dieser Pumpe und die Leitfähigkeitssensoren 9 und 10 dargestellt sind. Die Leitfähigkeitssensoren messen vorzugsweise die temperaturkorrigierte Leitfähigkeit der Dialysierflüssigkeit auf der Basis der Na-Konzentration. Anstelle der Bestimmung der Leitfähigkeit kann die Konzentrationsmessung auch über die Messung von entsprechenden optischen Eigenschaften erfolgen.

Der übrige Aufbau ist bekannt, wozu beispielsweise auf die eingangs zitierte EP 0 097 366 hingewiesen wird. Die Dialysierflüssigkeit durchströmt die Dialysatkammer 4 mit einer durch die Drehzahl der Pumpe 7 vorgegebenen Flußrate Qd sowie einer durch die nicht dargestellte Konzentratmischung vorgegebenen Eingangskonzentration cdi, die mittels des stromauf angeordneten Leitfähigkeitssensors 9 erfaßt wird. Die sich bei der Dialyse einstellende Ausgangskonzentration cdo wird mittels des stromab angeordneten Leitfähigkeitssensor 10 erfaßt. Aus der Differenz cdi - cdo wird der Elektrolyttransfer als Basisgröße für den zu bestimmenden Parameter berechnet. Prinzipiell kann der Leitfähigkeitssensor 9 entfallen und der Meßwert durch einen eingestellten, d.h. vorgegebenen Wert von cdi ersetzt werden.

Alle Signale für die Flüsse Qb und Qd sowie für die dialysatseitigen Konzentrationen cdi und cdo werden einer Auswertestufe 11 zugeführt, die vorzugsweise durch einen Mikroprozessor gebildet wird, der in der Regel ohnehin in einem Dialysegerät vorhanden ist. In dieser Auswertestufe 11 werden die Signale miteinander verknüpft, um den gewünschten Parameter der Hämodialyse zu bestimmen. So wird in dieser Stufe 11 u.a. die Elektrolyttransferrate Qd x (cdi - cdo) errechnet und mit anderen Größen u.a. auf der Basis der Massenbilanz im Dialysator in Beziehung gesetzt. Gemäß der Erfindung wird mindestens eine der beiden Flußraten Qb bzw. Qd, ausgelöst durch ein Steuersignal der Auswertestufe 11, über die Stufen 6 bzw. 8 auf mindestens zwei unterschiedliche Werte eingestellt, wobei von diesen Werten entsprechende Meßgrößen abgeleitet werden, die zusätzlich der Auswertestufe 11 zugeführt und dort mit anderen Größen verknüpft werden, um den zu bestimmenden Parameter zu ermitteln. Auf die Verknüpfung dieser Größen wird später noch eingegangen.

Vorzugsweise wird der Vollblutfluß Qvb mittels der Stufe 6 und der Blutpumpe 5 auf zwei unterschiedliche Werte Qvb1 und Qvb2 eingestellt. Maßgebend für die Dialyse ist dabei jedoch der effektive Blutfluß Qb. Dieser effektive Blutfluß wird aus dem Vollblutfluß Qvb nach bekannten Beziehungen abgeleitet (siehe Franz a.a.O). Diese Ableitung des Wertes Qb wird ebenfalls in der Auswertestufe 11 vorgenommen.

Alternativ oder auch zusätzlich kann der Dialysierflüssigkeitsfluß auf mindestens zwei unterschiedliche Werte Qd1 und Qd2 eingestellt und die davon abgeleiteten Signale in der Auswertestufe 11 mit anderen Größen verknüpft werden.

Vorzugsweise ist die Eingangskonzentration cdi der Dialysierflüssigkeit konstant. Es sind jedoch auch Fälle denkbar, bei denen im Meßintervall die Eingangskonzentration der Dialysierflüssigkeit innerhalb bestimmter Grenzen geändert wird, um einen weiteren Meßwert zu haben.

Im folgenden soll nunmehr die Verknüpfung der Meßgrößen und abgeleiteter Rechengrößen in der Auswertestufe erläutert werden; als rechnerisch zu bestimmender Parameter wurde einmal die Bluteingangskonzentration cbi bei gemessenem effektivem Blutfluß Qb und zum anderen der effektive Blutfluß bei bekanntem cbi gewählt.

Dabei werden ein Lösungsansatz und die zugehörigen Gleichungssysteme zur Ermittlung vorstehender Parameter dargestellt, die den allgemeinen Übergang eines Dialysators von einem ersten Zustand in mindestens einen anderen, d.h. von einem ersten Zustand eines Parameters mit erster Messung des Wertes dieses Parameters in mindestens einen zweiten mit zweiter Messung des Parameters, beschreiben. Das zugehörige Formelwerk ist so allgemein formuliert, daß zwischen den mindestens zwei Messungen bestimmte Parameter nicht mehr konstant gehalten werden müssen, solange sie bekannt sind bzw. gemessen werden oder bestimmte Parameterverhältnisse gegeben sind. In dem Lösungsansatz und den Gleichungssystemen sind daher auch alle Fälle, in denen sich konkret die Bluteingangskonzentration cbi, die Eingangskonzentration cdi der Dialysierflüssigkeit oder im Lösungsansatz verwendete Kenngrößen des Dialysators zwischen den beiden Messungen ändern, enthalten.

Der Lösungsansatz erfolgt über die Massenbilanz nach Gleichung (2), die in Beziehung gesetzt wird zu den Stoffaustausch bestimmenden Kenngrößen des Dialysators,

$$Qd \cdot (cdo\text{-}cdi) = Qb \cdot (cbi\text{-}cbo) = \frac{A \cdot \bar{c}_{\text{ln}}}{R} \qquad (4,5)$$

mit der Austauschfläche A des Dialysators, seinem spezifischen Membran-Diffusionswiderstand R und der im Gegenstromverfahren geltenden mittleren Konzentrationsdifferenz

$$\overline{c}_{ln} = \frac{(cbi - cdo) - (cbo - cdi)}{\ln\left(\dfrac{cbi - cdo}{cbo - cdi}\right)} \qquad (6)$$

Man kann Gl. (4) nach cbo auflösen und den gefundenen Ausdruck in (6) einsetzen:

$$\overline{c}_{ln} = \frac{(cbi - cdo) - \left[cbi - \dfrac{Qd\cdot(cdo - cdi)}{Qb} - cdi\right]}{\ln\left[\dfrac{cbi - cdo}{cbi - \dfrac{Qd\cdot(cdo - cdi)}{Qb} - cdi}\right]} = \frac{cdo\cdot\left(\dfrac{Qd}{Qb} - 1\right) + cdi\cdot\left(1 - \dfrac{Qd}{Qb}\right)}{\ln\left[\dfrac{cbi - cdo}{cbi - cdo\cdot\dfrac{Qd}{Qb} + cdi\cdot\left(\dfrac{Qd}{Qb} - 1\right)}\right]}$$

Damit ergibt sich:

$$\frac{\ln\left[\dfrac{cbi - cdo}{cbi - cdo\cdot\dfrac{Qd}{Qb} + cdi\cdot\left(\dfrac{Qd}{Qb} - 1\right)}\right]}{\left(\dfrac{Qd}{Qb} - 1\right)}\cdot Qd = \frac{A}{R} \qquad (7)$$

Der Gleichgewichtsübergang des Dialysatorss vom Zustand Z1 (Zahlen mit Index 1) in den Zustand Z2 (Zahlen mit Index 2) sei charakterisiert durch die Übergangskonstanten k1, k2, k3 mit

$$k1 := \frac{cbi2}{cbi1}$$

$$k2 := \frac{cdi2}{cdi1}$$

$$k3 := \frac{A2\cdot R1}{A1\cdot R2}$$

Es wird in Form der $k_i$ somit eine Aussage über die Änderung der drei Größen cbi, cdi, A/R gemacht, wenn das System vom Zustand Z1 in den Zustand Z2 übergeht.

Die Kenngröße A/R des Dialysators hängt - vor allem bei Highflux-Dialysatoren - von den Flüssigkeitsflüssen auf der Blut- und auf der Dialysatseite ab. In einem bestimmten Bereich der Flüsse Qd und Qb ist die Kenngröße konstant. Sie ist eine reproduzierbare Funktion von Qd und Qb, d.h. das erfindungsgemäße Verfahren kann auch bei Veränderung dieser Kenngröße angewendet werden, falls das Verhältnis beider Kenngrößen in den beiden Zuständen z.B. durch eine in-vitro-Messung bekannt ist und z.B. als Konstante oder als Funktionsausdruck fest in der Auswerteeinheit 11 abgelegt ist.

Damit kann beim Übergang des Systems über die Gleichung (7) gleichgesetzt werden:

$$\frac{\ln\left[\dfrac{cbi1 - cdo1}{cbi1 - cdo1\cdot\dfrac{Qd1}{Qb1} + cdi1\cdot\left(\dfrac{Qd1}{Qb1} - 1\right)}\right]}{\left(\dfrac{Qd1}{Qb1} - 1\right)}\cdot Qd1\cdot k3 = \frac{\ln\left[\dfrac{k1\cdot cbi1 - cdo2}{k1\cdot cbi1 - cdo2\cdot\dfrac{Qd2}{Qb2} + k2\cdot cdi1\cdot\left(\dfrac{Qd2}{Qb2} - 1\right)}\right]}{\left(\dfrac{Qd2}{Qb2} - 1\right)}\cdot Qd2 \quad ,$$

Zustand Z1(Qd1,Qb1,cdi1,cdo1,cbi1,A1,R1 →
Zustand Z2(Qd2,Qb2,cdi2,cdo2,cbi2,A2,R2)

Nach einer entsprechenden Umformung des Ausdrucks mit dem Ansatz: x·lna = lna* erhält man mit

$$q := \frac{Qd1}{Qb1}, \qquad n\cdot q := \frac{Qd2}{Qb2}$$

und damit

$$n = \frac{Qd2\cdot Qb1}{Qd1\cdot Qb2}$$

$$B := \frac{Qd1\cdot(n\cdot q - 1)}{Qd2\cdot(q - 1)}$$

die folgende Gleichung:

$$\left[\frac{k1\cdot cbi1 - cdo2}{(k1\, cbi1 - (cdo2\cdot n\cdot q) + (k2\cdot cdi\,(n\cdot q - 1)))}\right] - \left[\frac{cbi1 - cdo1}{cbi1 - cdo1\cdot q - (cdi1\cdot(q - 1))}\right]^{k3\cdot B} = 0$$

$$(9)$$

Die Gleichung (9) beschreibt generell den Stoffaustausch am Dialysator beim Gleichgewichtsübergang vom Zustand Z1 nach dem Zustand Z2, d.h. sie stellt für die beteiligten Größen eine Verbindung her zwischen den beiden Zuständen. Sie ist auf die unterschiedlichste Weise auswertbar. Soll zum Beispiel die Bluteingangskonzentration cbi bestimmt werden, wird bei gegebenen Meßwerten für alle Qd, Qb und cdi, cdo sowie bei gegebenen Konstanten in Gleichung (9) die Unbekannte cbi numerisch solange variiert, bis der Ausdruck = 0 ist, d.h. die Nullstelle der zugehörigen Funktion gefunden worden ist. Diese mathematische Maßnahme, die numerische Nullstellensuche, ist mit den heutigen Rechnern und einer entsprechenden Programmierung relativ schnell in der Auswertestufe 11 durchzuführen.

Es versteht sich, daß die Gleichung (9) nur eine bevorzugte mathematische Darstellung ist. Von der Erfindung werden jedoch alle damit gleichwirkenden Ausdrücke erfaßt, d.h. alle mathematischen Umformungen oder Näherungen der Gleichung (9).

Als Ergebnis der vorstehenden Auflösung der Gleichung (9) erhält man den Wert der Bluteingangskonzentration cbi. Ausgehend von diesem Wert können dann weitere Parameter der Hämodialyse, z.B. die Dialysance bzw. Clearance, bestimmt werden. Zur Bestimmung der beiden Werte für die Dialysance bei den beiden Flüssen wird die Gleichung (3) bzw. die adäquate Gleichung für Qb herangezogen. Auch dieser Rechenvorgang wird in der Stufe 11 vollzogen.

Die verschiedenen in der Gleichung (9) enthaltenen Parameter und Kenngrößen und einige vorteilhafte Einstellungen für die Auflösung der Gleichung sind nachfolgend aufgelistet.

- k1 bekannt, vorteilhaft = 1 (cbi ist konstant oder in bekannter Weise geändert).
- k2 bekannt, vorteilhaft = 1 (cdi ist konstant oder in bekannter Weise geändert).
- k3 bekannt, vorteilhaft = 1 (A/R ist konstant oder in bekannter Weise geändert).

- Qd1, Qd2 beide bekannt, besonders vorteilhaft mit Qd1=Qd2 .
- Qb1, Qb2 bekannt.
- cdo1, cdo2 bekannt.

Bei der Auflösung der Gleichung (9) nach cbi wird von zwei Blutflußeinstellungen ausgegangen, dem ersten, vorgegebenen Blutfluß Qb1 und dem zweiten, vorteilhafterweise niederigeren Blutfluß Qb2. Gemessen wird dabei i. a. jeweils der absolute Vollblutfluß Qvb. Diese Messung ist bekanntlich fehlerbehaftet. Erstens geht, wie bereits erwähnt, in die Bestimmung des effektiven Blutflusses Qb aus diesem Meßwert der Hämatokrit ein, der damit zusätzlich bestimmt werden muß. Zweitens ändert sich der Hämatokrit über die Dauer der Hämodialyse als Folge der Ultrafiltration.

Zusätzlich ist zu berücksichtigen, daß der Vollblutfluß nur annäherungsweise bekannt ist, da er üblicherweise über die Umdrehungsgeschwindigkeit der üblicherweise eingesetzten peristaltischen Blutpumpe und den Schlauchdurchmesser des Systems bestimmt wird. Da der Schlauchdurchmesser typischerweise nur mit einer Genauigkeit von +/- 5 % bekannt ist, und da weiterhin der Pumpensegmentquerschnitt durch den Ansaugunterdruck verringert werden kann, ergeben sich auch hierbei erhebliche Toleranzen.

Gemäß einer Weiterbildung der Erfindung läßt sich der effektive Blutfluß Qb aus der Gleichung (9) als gewünschter Parameter oder als Zwischenwert bestimmen, wenn der Wert cbi, die Bluteingangskonzentration, nach irgendeinem Verfahren, vorzugsweise nach der Bypass-Methode gemäß der Patentanmeldung 197 34 992.7 bekannt ist. Die Gleichung (9) ist dann nach Qb aufzulösen.

Damit bietet die erfindungsgemäße Vorrichtung nicht nur die Möglichkeit der Messung der Bluteingangskonzentration cbi und der in-vivo-Dialysance bzw. Clearance; es kann zusätzlich auch der absolute Blutwasseranteil bzw. der Anteil der zelligen Bestandteile, der Hämatokrit (HCT), im Blut gemessen werden, weil die mit Gleichung (9) bei bekanntem cbi ermittelten effektiven Blutflüsse Qb1, Qb2 verglichen werden können mit dem geförderten Pumpvolumen Qvb der Blutpumpe, welches ja nicht nur den wässrigen Anteil, sondern auch die festen Bestandteile enthält. Für den vereinfachten Fall, daß allein das Plasmawasser das Lösungsmittel für den jeweiligen Stoff darstellt, gelten die Beziehungen

$$HCT = 1 - \frac{Qb1}{Qvb1} = 1 - \frac{Qb2}{Qvb2} = 1 - \frac{Qb3}{Qvb3}$$

Damit ergeben sich vielfältige Möglichkeiten für den Bereich der physiologischen Dialyse. So ist es beispielsweise möglich, eine Veränderung des Blutvolumens des Patienten festzustellen. Eine derartige Veränderung des Blutvolumens führt bekanntlicherweise zu einer Änderung des Hämatokrit und des wässrigen Anteils des Blutes.

Voraussetzung ist allerdings hierbei, daß die Transporteigenschaften der Dialysemembran unverändert bleiben, d.h., daß es nicht zu einer teilweise Blockade der Membran kommt. Derartige Störungen der Hämodialyse sind jedoch mittels geeigneter Detektoren feststellbar.

Weiterhin kann mittels der Vorrichtung auf Veränderungen der Transporteigenschaften der Membran geschlossen werden, sofern andere Methoden zur Bestimmung der Veränderung des Blutvolumens vorhanden sind. Aus dem Vergleich der damit berechneten Änderungen der Clearance mit der gemessenen Änderung können somit Veränderungen der Transporteigenschaft der Membran bestimmt werden.

**Patentansprüche**

**1.** Vorrichtung zur in-vivo-Bestimmung von am Stoffaustausch beteiligten Parametern der Hämodialyse, mit einem Dialysator (1), der eine semi-permeable Membran (2) aufweist, die eine Blutkammer (3) von einer Dialysatkammer (4) trennt, von denen die Blutkammer (3) an einen extrakorporalen Kreislauf (I) angeschlossen ist, der eine Blutpumpe (5) mit einer zugehörigen Einrichtung (6) zum Einstellen der Pumpgeschwindigkeit und somit des Vollblutflusses (Qvb) aufweist und von denen die Dialysatkammer (4) an einen Dialysatkreislauf (II) angeschlossen ist, der eine Dialysatpumpe (7) mit einer zugehörigen Einrichtung (8) zum Einstellen der Pumpgeschwindigkeit und somit des Dialysierflüssigkeitsflusses (Qd) sowie eine Einrichtung zum Bereitstellen von Dialysierflüssigkeit mit einer Eingangskonzentration (cdi) aufweist, und in den zumindest stromab des Dialysators (1) ein Konzentrations-Meßfühler (10) zur Messung der Konzentration (cdo) am Ausgang des Dialysators (1) geschaltet ist, und mit einer Auswerteschaltung (11), auf die das Ausgangsmesssignal des Konzentrations-Meßfühlers (10) sowie ein Signal für den Wert der Dialysat-Eingangskonzentration (cdi) geschaltet sind, wobei mit der Auswerteschaltung (11) Steuersignale zum Einstellen der Blutpumpe (5) und/oder der Dialysatpumpe (7) auf mindestens eine zweite Pumpgeschwindigkeit (Qvb1, Qvb2; Qd1, Qd2) erzeugbar sind, **dadurch gekennzeichnet,**

**dass** die Auswerteschaltung (11) derart ausgebildet ist, dass aus den Ausgangs-Messsignalen (cdo1, cdo2) des Konzentrations-Meßfühlers (10) sowie dem Signal für den Wert der Dialysat-Eingangskonzentration (cdi1, cdi2) sowie aus Flußmeßgrößen, die von den eingestellten Werten des Vollblutflusses (Qvb1, Qvb2) oder Dialysierflüssigkeitsflusses (Qd1, Qd2) abgeleitet sind, auf der Grundlage der Gleichsetzung der Massenbilanz zwischen der aus dem Blut entfernten Stoffmenge und der in derselben Zeit in der Dialysierflüssigkeit abtransportierten Stoffmenge einerseits mit den Stoffaustausch des Dialysators beschreibenden Kenngrößen (A, R) andererseits der zu bestimmende Parameter ermittelbar ist.

2. Vorrichtung nach Anspruch 1, **dadurch gekennzeichnet, dass** die Auswerteschaltung (11) derart ausgebildet ist, dass der Vollblutfluß (Qvb) auf mindestens zwei unterschiedliche Werte (Qvb1, Qvb2) einstellbar ist und von dem jeweiligen Blutfluß ein Signal für den effektiven Blutfluß (Qb1, Qb2) ableitbar ist.

3. Vorrichtung nach Anspruch 1, **dadurch gekennzeichnet, dass** die Auswerteschaltung (11) derart ausgebildet ist, dass der Vollblutfluß (Qvb) auf mindestens zwei unterschiedliche Werte (Qvb1, Qvb2) einstellbar und von dem jeweiligen Blutfluß ein Signal für eine mit dem effektiven Blutfluß (Qb1, Qb2) zumindest im Messintervall verknüpfte Messgröße ableitbar ist.

4. Vorrichtung nach einem der Ansprüche 1 bis 3, **dadurch gekennzeichnet, dass** die Auswertschaltung (11) derart ausgebildet ist, dass der Dialysierflüssigkeitsfluß (Qd) auf mindestens zwei unterschiedliche Werte (Qd1, Qd2) einstellbar ist und jeweils eine entsprechende Messgröße für den Dialysierflüssigkeitsfluß ableitbar ist.

5. Vorrichtung nach einem der Ansprüche 1 bis 4, **dadurch gekennzeichnet, dass** die Einrichtung zum Bereitstellen von Dialysierflüssigkeit mit einer Eingangskonzentration (cdi) derart ausgebildet ist, dass die Eingangskonzentration (cdi) der Dialysierflüssigkeit konstant ist.

6. Vorrichtung nach einem der Ansprüche 1 bis 4, **dadurch gekennzeichnet, dass** die Einrichtung zum Bereitstellen von Dialysierflüssigkeit mit einer Eingangskonzentration (cdi) derart ausgebildet ist, dass im Meßintervall die Eingangskonzentration (cdi) der Dialysierflüssigkeit innerhalb bestimmter Grenzen veränderbar ist.

7. Vorrichtung nach einem der Ansprüche 2 bis 6, **dadurch gekennzeichnet, dass** die Auswerteschaltung (11) derart ausgebildet ist, dass der gewünschte Parameter aus den Werten des Blut- und Dialysierflüssigkeitsflusses (Qb, Qd), der Dialysierflüssigkeitseingangs- und ausgangskonzentration (cdi, cdo) und der Bluteingangskonzentration (cbi) sowie der Austauschfläche (A) und dem spezifischen Membran-Diffusionswiderstand (R) des Dialysators (1) nach der Beziehung

$$\left[ \frac{k1 \cdot cbi1 - cdo2}{(k1 \cdot cbi1 - (cdo2 \cdot n \cdot q) - (k2 \cdot cdi \cdot (n \cdot q - 1)))} \right] - \left[ \frac{cbi1 - cdo1}{cbi1 - cdo1 \cdot q - (cdi1 \cdot (q - 1))} \right]^{k3 \cdot B} = 0$$

mit

$$q := \frac{Qd1}{Qb1} \qquad n \cdot q := \frac{Qd2}{Qb2}$$

(*n = Verhältnis der Flußverhältnisse*)

$$B := \frac{Qd1 \cdot (n \cdot q - 1)}{Qd2 \cdot (q - 1)} \qquad n = \frac{Qd2 \cdot Qb1}{Qd1 \cdot Qb2}$$

und den Übergangskonstanten

$$k1 = \frac{cbi2}{cbi1} \qquad k2 = \frac{cdi2}{cdi1} \qquad k3 = \frac{A2 \cdot R1}{A1 \cdot R2}$$

bestimmbar ist.

8. Vorrichtung nach Anspruch 7, **dadurch gekennzeichnet, dass** die Auswerteschaltung (11) derart ausgebildet ist, dass die Bluteingangskonzentration (cbi) bestimmbar ist.

9. Vorrichtung nach Anspruch 8, **dadurch gekennzeichnet, dass** die Auswerteschaltung (11) derart ausgebildet ist, dass aus der ermittelten Bluteingangskonzentration (cbi) die Dialysance (D) oder die Clearance (K) des Dialysators (1) bestimmbar ist.

10. Vorrichtung nach Anspruch 7, **dadurch gekennzeichnet, dass** die Auswerteschaltung (11) derart ausgebildet ist, dass der effektive Blutfluß (Qb) bestimmbar ist.

11. Vorrichtung nach Anspruch 10, **dadurch gekennzeichnet, dass** die Auswerteschaltung (11) derart ausgebildet ist, dass aus dem ermittelten effektiven Blutfluß (Qb) und dem eingestellten Vollblutfluß (Qvb) der Hämatokrit (HCT) nach der Beziehung

$$HCT = 1 - \frac{Qb}{Qvb}$$

bestimmbar ist.

12. Vorrichtung nach einem der Ansprüche 1 bis 11, **dadurch gekennzeichnet, dass** der Konzentrations-Meßfühler (10) ein Leitfähigkeits-Meßfühler oder optische Meßfühler ist.

**Claims**

1. A device for the in vivo determination of the haemodialysis parameters involved in the substance exchange, with a dialyser (1), which has a semipermeable membrane (2) which separates a blood chamber (3) from a dialysate chamber (4), whereof the blood chamber (3) is connected to an extracorporeal circuit (I), which has a blood pump (5) with an accompanying device (6) for adjusting the pump speed and thus the full blood flow (Qvb), and whereof the dialysate chamber (4) is connected to a dialysate circuit (II), which has a dialysate pump (7) with an accompanying device (8) for adjusting the pump speed and thus the dialysing fluid flow (Qd) as well as a device for the preparation of dialysing fluid with an input concentration (cdi), and into which dialysate circuit a concentration measuring sensor (10) for measuring the concentration (cdo) at the outlet of the dialyser (1) is incorporated at least downstream of the dialyser (1), and with an evaluation circuit (11), to which the output measuring signal of the concentration measuring sensor (10) as well as a signal for the value of the dialysate input concentration (cdi) are switched, whereby there can be generated with the evaluation circuit (11) control signals for adjusting the blood pump (5) and/or the dialysate pump (7) to at least a second pump speed,
(Qvb1, Qvb2; Qd1, Qd2)
**characterised in that** the evaluation circuit (II) is designed in such a way that, on the basis of equating the mass balance between the amount of substance removed from the blood and the amount of substance transported away in the same time in the dialysing fluid on the one hand with the characterising quantities (A, R) describing the substance exchange of the dialyser on the other hand, the parameter to be determined can be ascertained from the output measuring signals (cdo1, cdo2) of the concentration measuring sensor (10) and the signal for the value of the dialysate input concentration (cdi1, cdi2) and from the measured flow quantities which are derived from the adjusted values of the full blood flow (Qvb1, Qvb2) or dialysing fluid flow (Qd1, Qd2).

2. The device according to claim 1, **characterised in that** the evaluation circuit (11) is designed in such a way that the full blood flow (Qvb) can be adjusted to at least two different values (Qvb1, Qvb2) and a signal for the effective blood flow (Qb1, Qb2) can be derived from the respective blood flow.

3. The device according to claim 1, **characterised in that** the evaluation circuit (11) is designed in such a way that the full blood flow (Qvb) can be adjusted to at least two different values (Qvb1, Qvb2) and a signal for a measured quantity linked to the effective blood flow (Qb1, Qb2) at least in the measurement interval can be derived from the respective blood flow.

4. The device according to any one of claims 1 to 3, **characterised in that** the evaluation circuit (11) is designed in such a way that the dialysing fluid flow (Qd) can be adjusted to at least two different values (Qd1, Qd2) and a corresponding measured quantity can be derived in each case for the dialysing fluid flow.

**5.** The device according to any one of claims 1 to 4, **characterised in that** the device for the preparation of dialysing fluid with an input concentration (cdi) is designed in such a way that the input concentration (cdi) of the dialysing fluid is constant.

**6.** The device according to any one of claims 1 to 4, **characterised in that** the device for the preparation of dialysing fluid with an input concentration (cdi) is designed in such a way that, in the measurement interval, the input concentration (cdi) of the dialysing fluid can be changed within specific limits.

**7.** The device according to any one of claims 2 to 6, **characterised in that** the evaluation circuit (11) is designed in such a way that the desired parameter can be determined from the values of the blood and dialysing-fluid flow (Qb, Qd), the dialysing fluid input and output concentration (cdi, cdo) and the blood input concentration (cbi) as well as the exchange area (A) and the specific membrane diffusion resistance (R) of the dialyser (1) according to the relationship

$$\left[\frac{k1 \cdot cbi1 - cdo2}{(k1 \cdot cbi1 - (cdo2 \cdot a \cdot q) + (k2 \cdot cdi \cdot (a \cdot q - 1)))}\right] - \left[\frac{cbi1 - cdo1}{cbi1 - cdo1 \cdot q - (cdi1 \cdot (q - 1))}\right]^{k3 \cdot B} \Rightarrow 0$$

with

$$q: = \frac{Qd1}{Qb1} \qquad n \cdot q: = \frac{Qd2}{Qb2}$$

*(n = ratio of flow relations)*

$$B: = \frac{Qd1 \cdot (n \cdot q - 1)}{Qd2 \cdot (q - 1)} \qquad n = \frac{Qd2 \cdot Qb1}{Qd1 \cdot Qb2}$$

and the transition constants

$$k1 = \frac{cbi2}{cbi1} \qquad k2 = \frac{cdi2}{cdi1} \qquad k3 = \frac{A2 \cdot R1}{A1 \cdot R2}$$

**8.** The device according to claim 7, **characterised in that** the evaluation circuit (11) is designed in such a way that the blood input concentration (cbi) can be determined.

**9.** The device according to claim 8, **characterised in that** the evaluation circuit (11) is designed in such a way that the dialysance (D) or the clearance (K) of the dialyser (1) can be determined from the ascertained blood input concentration (cbi).

**10.** The device according to claim 7, **characterised in that** the evaluation circuit (11) is designed in such a way that the effective blood flow (Qb) can be determined.

**11.** The device according to claim 10, **characterised in that** the evaluation circuit (11) is designed in such a way that the hematocrit (HCT) can be determined from the ascertained effective blood flow (Qb) and the adjusted full blood flow (Qvb) according to the relationship

$$HCT = 1 - \frac{Qb}{Qvb}$$

**12.** The device according to any one of claims 1 to 11, **characterised in that** the concentration measuring sensor (10) is a conductivity measuring sensor or an optical measuring sensor.

**Revendications**

**1.** Dispositif de détermination in vivo de paramètres d'hémodialyse participant à l'échange de substances, comprenant

un dialyseur (1) présentant une membrane semi-perméable (2) qui sépare un compartiment pour le sang (3) d'un compartiment pour le dialysat (4) dont le compartiment pour le sang (3) est raccordé à un circuit extracorporel (I) qui présente une pompe à sang (5) avec un mécanisme associé (6) pour régler la vitesse de pompage et donc du débit de sang complet (Qvb), et dont le compartiment pour le dialysat (4) est raccordé à un circuit de dialysat (II) qui présente une pompe à dialysat (7) avec un mécanisme associé (8) pour régler la vitesse de pompage et donc du débit de fluide de dialyse (Qd) ainsi qu'un mécanisme pour fournir du fluide de dialyse avec une concentration d'entrée (cdi), et dans lequel est connecté au moins en aval du dialyseur (1), un capteur de concentration (10) pour mesurer la concentration (cdo) à la sortie du dialyseur (1), et comprenant un circuit d'évaluation (11) auquel est connecté le signal de mesure de sortie du capteur de concentration (10) ainsi qu'un signal pour la valeur de la concentration d'entrée de dialysat (cdi), le circuit d'évaluation (11) pouvant produire des signaux de commande pour régler la pompe à sang (5) et/ou la pompe à dialysat (7) sur au moins une deuxième vitesse de pompage (Qvb1, Qvb2 ; Qd1, Qd2),

**caractérisé en ce que**

le circuit d'évaluation (11) est réalisé de telle sorte qu'il est possible de détecter le paramètre à déterminer à partir des signaux de mesure de sortie (cdo1, cdo2) du capteur de concentration (10) ainsi qu'à partir du signal pour la valeur de la concentration d'entrée de dialysat (cdil, cdi2) ainsi qu'à partir de grandeurs mesurées de débit, qui sont dérivées des valeurs réglées du débit de sang complet (Qvb1, Qvb2) ou du débit de fluide de dialyse (Qd1, Qd2), sur la base de l'assimilation du bilan de masse entre la quantité de matière éliminée du sang et de la quantité de matière évacuée en même temps dans le fluide de dialyse d'une part, avec les grandeurs caractéristiques (A, R) décrivant l'échange de substances du dialyseur d'autre part.

2. Dispositif selon la revendication 1, **caractérisé en ce que** le circuit d'évaluation (11) est réalisé de telle sorte que le débit de sang complet (Qvb) peut être réglé sur au moins deux valeurs différentes (Qvb1, Qvb2) et un signal pour le débit sanguin effectif (Qb1, Qb2) peut être dérivé à partir du débit sanguin respectif.

3. Dispositif selon la revendication 1, **caractérisé en ce que** le circuit d'évaluation (11) est réalisé de telle sorte que le débit de sang complet (Qvb) peut être réglé sur au moins deux valeurs différentes (Qvb1, Qvb2), et un signal pour une grandeur mesurée liée au débit de sang effectif (Qb1, Qb2) au moins dans l'intervalle de mesure peut être dérivé à partir du débit sanguin respectif.

4. Dispositif selon l'une quelconque des revendications 1 à 3, **caractérisé en ce que** le circuit d'évaluation (11) est réalisé de telle sorte que le débit de fluide de dialyse (Qd) peut être réglé sur au moins deux valeurs différentes (Qd1, Qd2), et une grandeur mesurée correspondante peut être dérivée respectivement pour le débit de fluide de dialyse.

5. Dispositif selon l'une quelconque des revendications 1 à 4, **caractérisé en ce que** le mécanisme de fourniture de fluide de dialyse avec une concentration d'entrée (cdi) est réalisé de telle sorte que la concentration d'entrée (cdi) du fluide de dialyse est constante.

6. Dispositif selon l'une quelconque des ) revendications 1 à 4, **caractérisé en ce que** le mécanisme de fourniture de fluide de dialyse avec une concentration d'entrée (cdi) est réalisé de telle sorte que dans l'intervalle de mesure, la concentration d'entrée (cdi) du fluide de dialyse est variable dans certaines limites.

7. Dispositif selon l'une quelconque des revendications 2 à 6, **caractérisé en ce que** le circuit d'évaluation (11) est réalisé de telle sorte que le paramètre souhaité peut être déterminé à partir des valeurs du débit de sang et de fluide de dialyse (Qb, Qd), de la concentration de fluide de dialyse d'entrée et de sortie (cdi, cdo) et de la concentration d'entrée de sang (cbi) ainsi que de la superficie d'échange (A) et de la résistance spécifique de diffusion de membrane (R) du dialyseur (1) selon la relation

$$\left(\frac{k1 \cdot cbi1 - cdo2}{(k1 \cdot cbi1 - (cdo2 \cdot n \cdot q) - (k2 \cdot cdi \cdot (n \cdot q - 1)))}\right)$$
$$- \left(\frac{cbi1 - cdo1}{cbi1 - cdo1 \cdot q - (cdi1 \cdot q(cdi1 \cdot (q - 1)))}\right)^{k3 \cdot B} = 0$$

où

$$q := \frac{Qd1}{Qb1} \qquad n \cdot q := \frac{Qd2}{Qb2}$$

(n = rapport du rapport de débit)

$$B := \frac{Qd1 \cdot (n \cdot q - 1)}{Qd2 \cdot (q - 1)} \qquad n = \frac{Qd2 \cdot Qb1}{Qd1 \cdot Qb2}$$

et les constantes de transition

$$k1 = \frac{cbi2}{cbi1} \qquad k2 = \frac{cdi2}{cdi1} \qquad k3 = \frac{A2 \cdot R1}{A1 \cdot R2}$$

8. Dispositif selon la revendication 7, **caractérisé en ce que** le circuit d'évaluation (11) est réalisé de telle sorte que la concentration d'entrée de sang (cbi) peut être déterminée.

9. Dispositif selon la revendication 8, **caractérisé en ce que** le circuit d'évaluation (11) est réalisé de telle sorte qu'il est possible de déterminer la dialysance (D) ou la clearance (K) du dialyseur (1) à partir de la concentration d'entrée de sang (cbi) détectée.

10. Dispositif selon la revendication 7, **caractérisé en ce que** le circuit d'évaluation (11) est réalisé de telle sorte que le débit sanguin effectif (Qb) peut être déterminé.

11. Dispositif selon la revendication 10, **caractérisé en ce que** le circuit d'évaluation (11) est réalisé de telle sorte qu'il est possible de déterminer l'hématocrite (HCT) à partir du débit sanguin effectif (Qb) détecté et du débit de sang complet (Qvb) réglé, selon la relation

$$HCT = 1 - \frac{Qb}{Qvb}$$

12. Dispositif selon l'une quelconque des revendications 1 à 11, **caractérisé en ce que** le capteur de concentration (10) est un capteur de conductibilité ou un capteur optique.